Europäisches Patentamt

(19) European Patent Office

Office européen des brevets

(11) Publication number: **0 232 596 B1**

# EUROPEAN PATENT SPECIFICATION

(12)

(45) Date of publication of patent specification:
**16.01.91 Bulletin 91/03**

(51) Int. Cl.⁵ : **A61M 35/00, A61B 19/00,
B65D 47/42**

(21) Application number: **86308960.3**

(22) Date of filing: **17.11.86**

---

(54) **Liquid applicator.**

(30) Priority: **18.11.85 US 798989**

(43) Date of publication of application:
**19.08.87 Bulletin 87/34**

(45) Publication of the grant of the patent:
**16.01.91 Bulletin 91/03**

(84) Designated Contracting States:
**DE FR GB IT SE**

(56) References cited:
**FR-A- 1 086 705
FR-A- 1 205 411
GB-A- 2 119 235
US-A- 4 183 684
US-A- 4 189 245
US-A- 4 229 116
US-A- 4 463 880**

(73) Proprietor: **MINNESOTA MINING AND
MANUFACTURING COMPANY
3M Center, P.O. Box 33427
St. Paul, Minnesota 55133-3427 (US)**

(72) Inventor: **Wirt, David F. c/o Minnesota Mining
and
Manufacturing Company 2501 Hudson Road
P.O. Box 33427 St.Paul Minnesota 55133 (US)**

(74) Representative: **Baillie, Iain Cameron et al
c/o Ladas & Parry Isartorplatz 5
D-8000 München 2 (DE)**

# Description

## Technical Field

This invention relates to liquid applicators having particular utility in the field of aseptic surgery preparation. More particularly, this invention relates to articles useful in applying pre-operative surgical scrubs or paints to skin.

## Background of Invention

Antiseptic preparation of patients for surgery conventionally includes a 3-10 minute scrubbing of the affected area with a soap solution followed by the application of a water-soluble antiseptic paint solution. These solutions are generally applied with saturated sponges that are attached to a blade or held with forceps. The sponges are saturated by soaking in open pans of solution. Numerous devices have been developed in an attempt to prevent the dripping of solution associated with the original technique. Another goal was to reduce the time required for application of the antiseptic solution.

The most important problem associated with prior art devices developed to apply surgical prep solutions is the lack of control of the delivery of the fluid to the sponge in a manner that will prevent dripping. Additional problems encountered with such devices include manufacturing complexity, reliability of rupturable joints and inappropriate geometries.

U.S. Patent Nos. 4,415,288 ; 4,507,111 ; and 4,498,796 describe a device that includes a liquid-containing rupturable cylindrical cartridge which is slidable within a tubular handle having one or two hollow interior spikes. In the device of U.S. Patent No. 4,498,796, the spikes communicate to recesses bored or burned into the interior of the sponge to facilitate free flow and even distribution of the fluid. In order for fluid to flow by gravity from this device, air must be entrained into the cartridge through the sponge and at least one hollow spike. For the following two reasons, this is not a reliable and predictable means to control the rate of delivery of fluid to the sponge. First, as the sponge becomes wet its air permeability decreases thereby restricting the entrainment of air. Second, hollow tubes with sufficiently small inside diameters to effectively meter the fluid flow rate to prevent dripping tend to "air-lock" due to the surface tension of the liquid. An additional disadvantage of the design of this device is the recesses in the sponge. These recesses are an attempt to improve the predictability of air entrainment and fluid distribution, but add to the manufacturing costs.

The device described in U.S. Patent 3,847,151 includes a sponge mounted on a nozzle extending from a hollow handle which contains an antiseptic solution. The solution is dispensed into the sponge when a rupturable joint in the nozzle is broken and external pressure is applied to the flexible handle. The problems associated with the design of this device include the unreliability of the rupturable joint and the lack of control of the fluid delivery into the sponge. Inherent mass production variability makes the fabrication of a reliable rupturable joint based on stress concentration difficult. After the nozzle is opened, it is very difficult, in practice, to deliver precisely the correct volume of fluid to saturate the sponge without dripping. In addition, as the sponge is wetted by the fluid, its ability to entrain air is diminished.

U.S. Patent 4,148,318 describes a device that includes an antiseptic solution contained in an integral reservoir with a frangible cover. Spikes affixed to a recess in the sponge pierce the cover to release the solution. The disadvantage of this device and other devices that include liquid containing ampoules, such as that described in U.S. Patent No. 3,891,331, is the absence of means to control the delivery of fluid to the sponge.

Another prior art is that disclosed in US-A-4,183,684 in which a fluid dispensing unit has a housing carrying a frangible fluid-containing ampoule, there being a layer of metering material disposed over a dispensing end of the unit and a layer of sponge material such as open-celled foam disposed over the exterior surface of said layer of metering material. The metering material is of a non-woven nylon scrim or a cotton or cotton based material. Cotton gauze and nylon scrim materials have reservoiring capacities in excess of about 5.0 cc/gm resulting in a fluid waste since this is not available for application to a surface to be treated.

FR-A- 1,205,411 discloses a container holding a supply of liquid and having a circular dispensing opening over which is disposed a first disc of porous material to hold therein a certain dose of the liquid when the container is inverted. Downstream of this first disc, and preferably spaced therefrom, is a second disc that is preferably of the same material as the first disc. Both discs are of a spongy porous material. On the outer face of the second disc there can be a covering layer of a knitted material or a cloth of synthetic material. In use the device is pressed against the skin with a pressure sufficient to cause the second disc to abut against the first disc. The amount of liquid applied to the skin is variable depending upon the manual pressure that is exerted in squeezing the first and second discs together. US-A- 4,463,880 is concerned with a squeeze bottle type dispenser having a hydrophobic and microporous antibacterial filter secured over a dispensing outlet. This filter may be of a fabric composed of polycarbonate (with or without glass fiber reinforcement) to provide a pore size in the range of 0.2 micron. In use the bottle must be squeezed to force liquid through the filter. In this prior art the rate of liquid dispensation is not regulated by the device itself, the rate being dependent upon the force exerted by the user.

In all of the above the absence of specific means to control the fluid flow rate to the sponge limits the volume of solution that can be delivered without dripping.

U.S. Patent No. 4,342,522 describes a roll-on dispenser which includes a porous open-cell foam membrane deformable by an applicator ball to regulate the dispensation of controlled amounts of powders. The amount of material dispensed is dependent upon the porosity of the membrane and the porosity of the mambrane is dependent upon the degree of its deformation by the ball upn operation of the dispenser.

Summary of the Invention

This invention relates to an article useful as a dispenser for the application of a liquid to a surface comprising

(a) a hollow elongate member having a major orifice at one end ;

(b) a layer of unfoamed porous metering material disposed over the major orifice of the hollow elongate member, the porous metering material is rigid, has a reservoiring capacity of less than about 5.0 cc/g and the layer is capable of regulating the flow of liquid therethrough ;

(c) a layer of sponge material disposed over the exterior surface of the layer of porous metering material ; and

(d) an air vent in the hollow elongate member capable of providing air flow between the exterior and interior of the hollow elongate member.

The current invention provides a means to reliably deliver in a short period of time a surgical prep solution to an applicator sponge without dripping. The applicator of the present invention controls the flow rate of liquid therein to the applicator sponge without the need for external operator manipulation. Unlike the devices of the prior art, the applicator of this invention provides control of the flow rate of liquid to the applicator sponge without squeezing the liquid container or compressing the applicator sponge. Although intended to apply modern, low viscosity, non-water soluble, film-forming prep solutions, this device can be configured to apply a variety of solution compositions, viscosities and volumes without compromising the fast wetting and no dripping features. The geometry of the device was designed to be compatible with modern antiseptic techniques. Because fabrication of the device utilizes high volume production processes, the manufacturing costs of the device are sufficiently inexpensive to permit disposable use.

Brief Description of the Drawings

Figure 1 is a perspective view of a preferred applicator of this invention ;

Figure 2 is a cross-section side view of the various unassembled elements of the preferred applicator of Figure 1, excluding reservoir 1 ;

Figure 3 is a cross-section auxiliary view of the top portion of the hollow elongate member shown in Figure 2 ;

Figure 4 is a cross-section of the dispensing end of the assembled applicator of Figure I ; and

Figure 5 is a perspective view of a preferred porous metering insert.

Detailed Description of the Invention

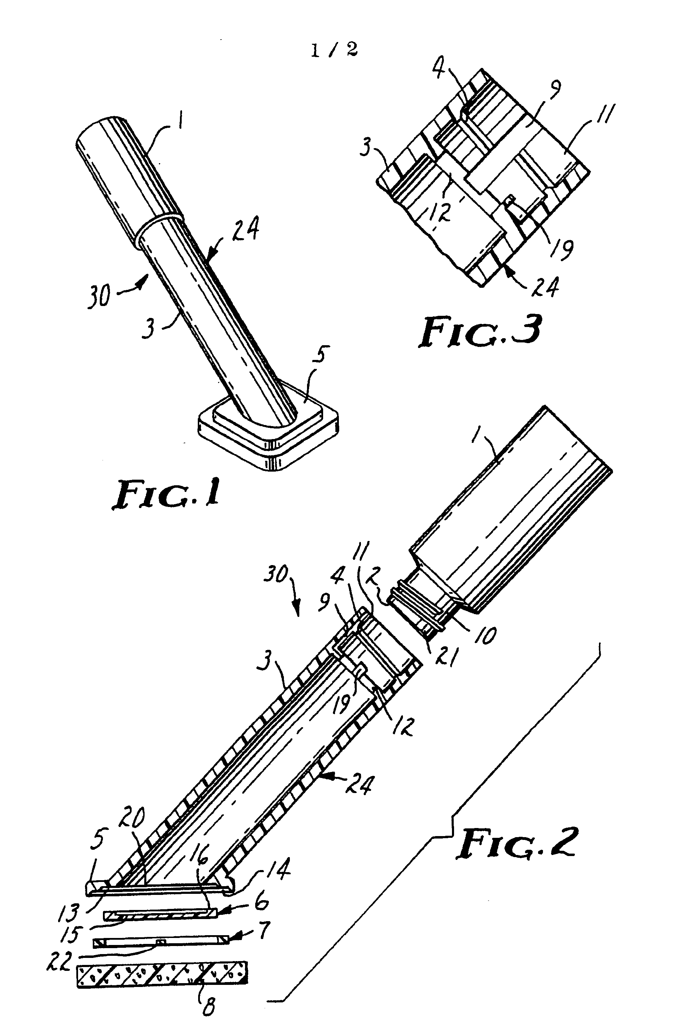

With reference to Figures 1 and 2, the applicator 30 includes a reservoir 1 containing the solution to be applied by the applicator, and a hollow elongate member 24 which acts as a handle and a conduit through which the solution passes before it is dispensed by foam sponge 8. Preferably, hollow elongate member 24 is bounded by major orifice 11 at one end and flange 5 at the opposite end. It is constructed so as to include a fixation means 4 adjacent orifice 11 for engaging at orifice 11 reservoir 1, a tubular handle 3 communicating between major orifice 11 and major orifice 20 defined by flange 5, and an air vent 9. Air vent 9 aspirates air into hollow elongate member 24 as liquid flows out through major orifice 20 during the dispensation process, thereby maintaining atmospheric pressure inside the applicator.

A substantially rigid porous metering insert 6 is disposed over major orifice 20 and is sandwiched between the underside of flange 5 and open-cell foam sponge 8. The porous metering insert 6 is disposed over major orifice 20 to control the flow of liquid out of hollow elongate member 24 and into foam sponge 8.

Foam sponge 8 can be selected from a variety of commercially available materials having a wide range of compression set ratios (i.e., density) and porosities. By varying the pore size, void fraction and hydrophilicity of the porous metering insert 6 and the compression set ratio and porosity of the foam sponge 8, applicators can be constructed to apply a variety of solution compositions, viscosities and volumes. The pore size, void volume fraction, and hydrophilicity of porous metering material 6 and the compression set ratio and porosity of open-cell foam sponge 8 are adjusted in relation to the viscosity, volume and surface tension of the liquid to be dispensed to allow a portion of the liquid contained in the applicator to flow to the outer surface of sponge 8 but not to allow the liquid to drip from the open-cell foam sponge 8 when it is suspended in mid-air with flange 5 down. It should be noted that to create a completely dripless applicator the amount of liquid to be dispensed by the device must not exceed the reservoiring capacity of sponge 8.

Metering insert 6 and sponge 8 are held together and disposed over major orifice 20 by a variety of suitable means, including adhesive bonding. In the preferred embodiment, elongate member 24 includes a flange 5 surrounding major orifice 20. The inner portion of flange 5 contains two lips defined by recesses 13 and 14. Metering insert 6 is contained within recess 13 and is held therein by foam sponge insert 8 which is adhered to the outer rim of flange 5 by heat-activatable bonding insert 7.

The invention will now be more particularly described in terms of the following preferred embodiment.

Figure 1 shows the preferred embodiment fully assembled from each of the parts shown in Figure 2. The device illustrated in Figure 1 consists of the following components as shown in Figure 2 : reservoir 1 containing the prep solution and sealed at its major orifice 21 by seal 2 ; hollow elongate member 24 being threaded at one end to accept reservoir 1, and having an angled flange 5 at the opposite end, and including air vent 9 ; substantially rigid porous metering insert 6 disposed within flange 5 ; thermoplastic bonding insert 7 also disposed within flange 5 ; and foam sponge 8.

Reservoir 1 is preferably a bottle with a threaded opening molded from a thermoplastic material compatible with the prep solution. The preferred embodiment comprises an essentially rigid, high-density polyethylene, cylindrical bottle with threaded portion 10 smaller in diameter than the main body of the bottle. Reservoir 1 is preferably filled and inductively sealed with foil seal 2 using conventional techniques.

Hollow elongate member 24 can be molded from any thermoplastic compatible with the liquid to be dispensed. Preferably, hollow elongate member 24 is molded from medium density polyethylene. Features of the preferred embodiment of this component include tubular handle 3, major orifice 11 which communicates with reservoir 1 at orifice 21 thereof, integrally-formed radially-projecting interior flange 12 which acts as a reservoir seat, integrally-formed fixation means 4 which threadably engages reservoir 1, projecting means 19 to tear seal 2 on reservoir 1, air vent 9, and flange 5 located at the opposite end of hollow elongate member 24 from orifice 11.

For surgical prep applications it is important that tubular handle 3 be long enough to prevent contact of the patient by the person applying the surgical prep solution. Preferably for such applications tubular handle 3 is at least about four inches long.

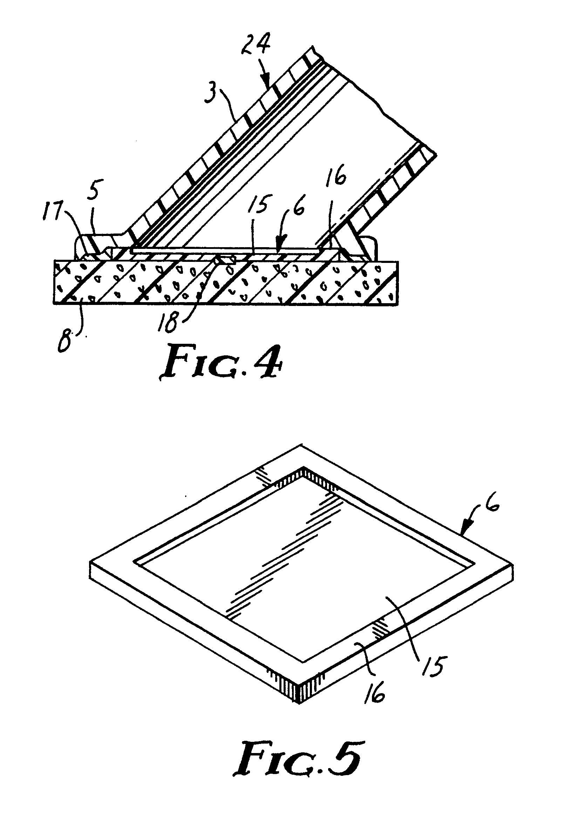

Projecting means 19 is positioned within hollow elongate member 24 so as to be capable of puncturing seal 2 on reservoir 1 when reservoir 1 is engaged at orifice 11 by fixation means 4. Many different geometries are possible for projecting means 19. The preferred embodiment, as shown in Figure 3, includes an integrally-formed projection that first extends radially from integrally-formed radially-projecting interior flange 12 and then extends in a direction towards orifice 11, parallel to the longitudinal axis of elongate member 24. The projection 19 is preferably of a size sufficient to begin rupturing seal 2 after reservoir 1 has been threaded at least one-half turn into hollow elongate member 24, with complete rupturing occurring three-fourth turn thereafter. Other possible embodiments of projecting means 19 include a hollow metal or polymeric spike.

Air vent 9 is a necessary component of the applicator. Metering insert 6 greatly restricts the amount of air which can enter the applicator through orifice 20. In order to avoid creating a vacuum and restricting flow through the device a means of maintaining atmospheric pressure in the device must be employed. Air vent 9 provides such a means. Air vent 9 is preferably an L-shaped slot having its long leg lying parallel to the longitudinal axis of elongate member 24 and recessed through the threads of fixation means 4, and its short leg lying perpendicular to the longitudinal axis of elongate member 24 and recessed through an upper portion of interior flange 12. Preferably, air vent 9 is located on the wall of hollow elongate member 24 which forms an obtuse angle with flange 5. In this manner, the liquid runs down the side of tubular handle 3 which is furthest from air vent 9 when the applicator is inverted in the position shown in Figure I. This location of air vent 9 minimizes the potential for leakage when the applicator is moved into position for use.

Further, the short leg of air vent 9 projects only through an upper portion of flange 12, leaving the lower portion of this flange intact and radially projecting into the interior of elongate member 24. In this manner when the applicator is inverted from the position shown in Figure 1 and the liquid is allowed to flow from tubular handle 3 back into reservoir 1, the liquid cascades over flange 12 and past air vent 9, thereby minimizing the potential for leakage of liquid through air vent 9. As a further means of reducing the potential of leakage, the size of air vent 9 is minimized.

Other geometries and locations of air vent 9 are envisioned by the inventor. For example the air vent could merely comprise a hole through tubular handle 3. Preferably such a hole would not be present in a location which would result in leakage of liquid from the device.

Preferably flange 5 is integrally formed and is angled from the longitudinal axis of elongate member 24 by between about 30 and 90 degrees. Most preferably there is about a 45 degree angle between flange 5 and the longitudinal axis of elongate member 24. Flange 5 preferably includes recesses 13 and 14 on its interior surface. Recesses 13 and 14 are dimensioned and shaped to permit nesting therein of metering insert 6 and bonding insert 7, respectively. Preferably recess 14 is greater in area than recess 13 to provide for sandwiching of metering insert 6 between orifice 20 and bonding insert 7.

The distribution and rate of delivery of the liquid to open-cell foam sponge 8 is controlled by the substantially rigid porous metering insert 6. For a given volume, viscosity, and surface tension of the liquid, rapid wetting of the foam sponge without dripping can be accomplished by appropriate specification of the average pore size, void volume fraction and hydrophilicity of porous metering insert 6 and the permanent compression set ratio and porosity of open-cell foam sponge 8.

Preferably, the pore size, void volume and hydrophilicity of porous metering insert 6 are adjusted so that for any given volume, viscosity and surface tension of the liquid to be applied the average flow rate of the liquid through metering insert 6 is between about 0.25 and 10 ml/sec. Greater flow rates than this will tend to result in an applicator which drips, while lower flow rates will result in an applicator which does not provide adequate liquid for surgical scrub applications. Most preferably for surgical scrub applications, the average flow rate of liquid through porous metering insert 6 is between 1 and 5 ml/sec. In general, for lower volumes and/or higher viscosity liquids the pore size and/or void volume of porous metering insert 6 is adjusted upwards to achieve the desired flow rates. Furthermore, as the surface tension of the liquid increases the metering insert is varied from a hydrophobic to a hydrophilic material to achieve the desired flow rate.

Porous metering insert 6 is substantially rigid and can be constructed from either hydrophilic or hydrophobic materials depending upon the liquid to be dispensed. Metering insert 6 can be in the form of sintered metal or plastic, molded unfoamed porous plastic, porous plastic films, porous metal structures and porous ceramics. Methods for making such porous structures include well-known sintering or leaching processes. Preferably, porous metering inserts are constructed from ceramics or thermoplastic materials such as polypropylene, polyethylene, polyvinylidene fluoride, ethylene-vinyl acetate copolymers, styrene-acrylonitrile copolymers and polytetrafluoroethylene. The hydrophilicity of the metering insert can be increased by various well-known treatments.

In general, the average pore size of metering insert 6 is between about 1 and 2,000 microns and the void volume is between about 10 and 70 percent. Preferably, for surgical applications where antiseptic solutions are dispensed, the average pore size of the metering insert is between about 10 and 500 microns and the void volume is between about 20 and 60 percent. Most preferably for such applications the average pore size is between about 60 and 100 microns and the void volume is between about 30 and 50 percent. A preferred ceramic material for use as a porous metering insert is 3M Brand Porous Structures™, commercially available from 3M, Saint Paul, Minnesota. This material has an average pore size of between about 14 and 175 microns and a void volume of approximately 30 percent. A particularly preferred porous metering material is a porous high density polyethylene commercially available as Interflo® Porous Plastic, F/N35-160-22, from Chromex Chemical Corp., Brooklyn, New York. This material has an average pore size of between about 60 and 100 microns and a void volume of between about 40 and 60 percent. In general, metering insert 6 is relatively non-reservoiring, having a reservoiring capacity of less than about 5.0 cc/g, preferably less than about 1.0 cc/g, and most preferably about 0.7 cc/g. Fluid which is reservoired in an intermediate flow regulator is not available for application to the surface to be treated. Thus, metering insert 6 provides controlled flow of fluid without significant fluid waste due to retained fluid within the metering insert itself.

As shown most clearly in Figures 4 and 5, the preferred embodiment of porous metering insert 6 has an area which is greater than the area of major orifice 20. Preferably, the area of insert 6 is at least two times greater than the area of orifice 20, and is most preferably almost as large as foam sponge 8. The preferred embodiment of metering insert 6 also has a raised periphery 16 which extends above planar metering area 15 and creates a cavity between active metering area 15 and major orifice 20. Preferably, raised periphery 16 extends above planar metering area 15 to the extent of one to two times the thickness of planar metering area 15. Both the increased area of insert 6 and raised periphery 16 serve to increase the area of fluid distribution to open-cell foam sponge 8.

Bonding insert 7 is a gasket-like structure, preferably having a rectangular annular shape. Preferably a bridging member 22 is included between opposing sides of bonding insert 7 to provide stuctural support to foam sponge 8 and prevent sagging thereof due to the weight of the absorbed fluid to be dispensed.

With reference to Figure 4, after assembly of the applicator and inductive melting of bonding insert 7, metering insert 6 and foam sponge 8 are peripherally affixed to flange 5 by bond 17, and metering insert 6 and foam sponge 8 are bonded together at bond 18.

Bonding insert 7 can be molded, extruded or die cut from a compatible thermoplastic or heat-activatable material filled with an inductively active material. Examples include laminated composites comprised of metal foil sandwiched between layers of thermoplastic or heat-activatable bonding material, such as polyethylene or hot-melt adhesives. Preferred examples include such thermoplastic or heat-activatable bonding materials filled with an inductive metal or metal oxide powder, e.g., 5 to 50 percent by volume iron powder having an average particle size of from about 57-72 µm (300 to 400 mesh). A particularly preferred bonding insert 7 is a rigid gasket formed from injection molded polyethylene filled with about 12 to 15 percent by volume iron powder, commercially available from Emabond Inc., Norwood, New Jersey, as G-10-205.

Open-cell foam sponge 8 comprises an open-cell foam material compatible with the liquid to be dispensed. Suitable open-cell foam sponge materials are prepared from elastomeric thermoplastic materials such as polyethylene and polyurethane. Especially preferred open-cell foam materials are prepared from polyurethane elastomers.

By utilizing a permanently compression-set foam, the wicking and reservoiring properties of the foam sponge 8 can be selected such that the fluid delivered through metering insert 6 wets foam sponge 8 but does not drip from sponge 8 when it is suspended in mid-air with flange 5 down. The greater the compression set ratio, the greater the amount of liquid that can be absorbed by the sponge material. Preferably, the foam sponge material is compression set (i.e., compressed) by heat and pressure to from about 1.5 to 10 times its original density, i.e., the compression set ratio is between about 1.5 and 10. Most preferably the compression set ratio of sponge 8 is between about 2 and 4.

The porosity of the foam sponge material can be selected such that foam sponge 8 will release a uniform amount of liquid when it is lightly rubbed against the surface upon which the liquid is to be dispensed. Preferably, for surgical prep applications, the porosity of the foam sponge material is between about 10 and 100 pores per linear 2,54 cm, more preferably about 90 pores per linear 2,54 cm. A particularly preferred open-cell foam sponge material is an elastomeric polyurethane foam having a compression set ratio of between about 2 and 4 and a porosity of about 90 pores per linear 2,54 cm, commercially available from Scotfoam Corp., Eddystone, Pennsylvania, as Scottfelt™ 3-900-Z.

The applicator of this invention is useful in dispensing liquids having viscosities at ambient temperatures of less than about 10,000 cps, most preferably less than about 500 cps.

As noted above, the applicator is useful in dispensing antiseptic liquids to cleanse a surgical field prior to surgery. Examples of suitable antiseptic preparations include those described in U.S. application Serial No. 617,255, filed June 4, 1984, and those described in U.S. 4,542,012, the disclosures of which are incorporated herein by reference. The antiseptic liquid is placed in the applicator and the applicator head is gently rubbed over the surgical field to thereby cleanse it. The applicator can be handled easily without dripping the liquid onto other articles in the vicinity of the surgical field. The dripless feature allows application of the liquid to only the desired areas of the patient and also allows for quicker and more efficient use of a surgical facility due to elimination of the time needed to clean the facility of the drippings of antiseptic prep.

## Claims

1. Dispenser (30) for the application of a liquid to a surface comprising :
   (a) a hollow elongate member (24) having a major orifice (20) at one end thereof.
   (b) a layer of unfoamed porous metering material (6) disposed over said major orifice (20) ;
   (c) a layer of sponge material (8) disposed over the exterior surface of said layer of porous metering material (6) ; and
   (d) an air vent (9) in said hollow elongate member (24) capable of providing air flow between the exterior and interior of said hollow elongate meber (24) ; **characterized in that** said porous metering material (6) is rigid,has a reservoiring capacity of less than about 5.0 cc/g and is capable of regulating the flow of liquid therethrough.

2. An applicator (30) in accordance with Claim 1 further characterized in that said porous metering material (6) is capable of limiting liquid flow thereghrough to between about 0.25 and 10 ml/sec.

3. An applicator (30) in accordance with Claim 2 further characterized in that the average pore size of said porous metering material (6) is between about 1 and 2,000 microns and the void volume of said porous metering material (6) is between about 10 and 70 percent.

4. An applicator (30) in accordance with Claim 2 further characterized in that said porous metering material (6) is a high density polyethylene having an average pore size of between about 60 and 100 microns and a void volume of between about 40 and 60 percent.

5. An applicator (30) in accordance with Claim 1 further characterized in that said applicator further comprises a reservoir (1) containing the solution to be applied by said applicator, which reservoir (1) is contained within or communicates with said hollow elongate member (24).

6. An applicator (30) in accordance with Claim (5) wherein said hollow elongate member (24) comprises a second major orifice (11) at the end opposite said first major orifice (20) and a fixation means (4) formed on the interior of said hollow elongate member (24) adjacent said second major orifice (11), said fixation means (4) capable of engaging said reservoir (11) ; and a flange (12) projecting radially into the interior of said hollow elongate member (24), said flange (5) located adjacent said fixation means (4) and spaced from said second major orifice (11) by said fixation means (4) ; and wherein said air vent (9) comprises an L-shaped slot having one leg recessed through said fixation means (4) and the other leg recessed through an upper portion of said flange (12).

7. An applicator (30) in accordance with Claim 1 wherein said sponge material (8) has a compression set ratio of from about 1.5 to 10, and a porosity of between about 10 and 100 pores per linear 2.54 cm.

**Ansprüche**

1. Ausgabevorrichtung (30) zum Auftragen einer Flüssigkeit auf eine Fläche mit

(a) einem langgestreckten hohlen Glied (24), das an seinem einen Ende eine weite Öffnung (20) besitzt;

(b) einer über der weiten Öffnung (20) angeordneten Schicht aus einem nichtgeschäumten porösen Dosierwerkstoff (6);

(c) einer über der Außenfläche der Schicht aus dem porösen Dosierwerkstoff (6) angeordneten Schicht aus einem Schwammstoff (8); und

(d) einem in dem langgestreckten hohlen Glied (24) vorgesehenen Luftdurchlaß (9), durch den Luft zwischen der Außenseite und dem Innern des langgestreckten hohlen Gliedes (24) hindurchströmen kann;

dadurch gekennzeichnet, daß der poröse Dosierwerkstoff (6) starr ist und eine Speicherkapazität von weniger als etwa 5,0 cm³/g hat und fähig ist, das Strömen von Flüssigkeit durch ihn zu regulieren.

2. Auftragevorrichtung (30) nach Anspruch 1, dadurch gekennzeichnet, daß der poröse Dosierwerkstoff (6) fähig ist, die Strömung von Flüssigkeit durch ihn zwischen etwa 0,25 und 10 ml/s zu begrenzen.

3. Auftragevorrichtung (30) nach Anspruch 2, dadurch gekennzeichnet, daß die durchschnittliche Porengröße des porösen Dosierwerkstoffes (6) zwischen etwa 1 und 2000 µm und das Porenvolumen des porösen Dosierwerkstoffes (6) zwischen etwa 10 und 70% beträgt.

4. Auftragevorrichtung (30) nach Anspruch 2, dadurch gekennzeichnet, daß der poröse Dosierwerkstoff (6) ein Polyethylen hoher Dichte mit einer durchschnittlichen Porengröße zwischen etwa 60 und 100 µm und einem Porenvolumen zwischen etwa 40 und 60% ist.

5. Auftragevorrichtung (30) nach Anspruch 1, dadurch gekennzeichnet, daß die Auftragevorrichtung ferner einen Vorratsbehälter (l) aufweist, der die von der Auftragevorrichtung aufzutragende Lösung enthält und der in dem langgestreckten hohlen Glied (24) enthalten ist oder mit ihm in Verbindung steht.

6. Auftragevorrichtung (30) nach Anspruch 5, dadurch gekennzeichnet, daß das langgestreckte hohle Glied (24) an dem der ersten weiten Öffnung (20) entgegengesetzten Ende eine zweite weite Öffnung (11) besitzt und auf der Innenwandung des langgestreckten hohlen Gliedes (24) im Bereich der zweiten weiten Öffnung (11) mit einer Festlegeeinrichtung (4) ausgebildet ist, die zum Angriff an dem Vorratsbehälter (11) geeignet ist, daß in das Innere des langgestreckten hohlen Gliedes (24) ein Flansch (12) radial vorspringt, der im Bereich der Festlegeeinrichtung (4) angeordnet ist und von der Festlegeeinrichtung (4) im Abstand von der zweiten weiten Öffnung (11) gehalten ist, und daß der Luftdurchlaß (9) von einem L-förmigen Langloch gebildet wird, dessen einer Schenkel eine die Festlegeeinrichtung (4) durchsetzende Vertiefung und dessen anderer Schenkel eine einen oberen Teil des Flansches (12) durchsetzende Vertiefung bildet.

7. Auftragevorrichtung (30) nach Anspruch 1, dadurch gekennzeichnet, daß der Schwammstoff (8) ein Verhältnis von Druckverformung zu Druckverformungsrest von etwa 1,5 bis 10 und eine Porosität zwischen etwa 10 und 100 Poren pro lineare 2,54 cm hat.

**Revendications**

1. Distributeur (30) pour l'application d'un liquide à une surface, comprenant:

(a) un organe allongé creux (24) comportant un orifice principal (20) à une extrémité;

(b) une couche de matière de dosage poreuse non expansée (6) disposée sur ledit orifice principal (20);

(c) une couche de matière spongieuse (8) disposée sur la surface extérieure de ladite couche de matière de dosage poreuse (6); et

(d) un évent d'air (9) prévu dans ledit organe allongé creux (24) et capable d'assurer un écoulement d'air entre l'extérieur et l'intérieur dudit organe allongé creux (24);

caractérisé en ce que

ladite matière de dosage poreuse (6) est rigide, possède une capacité de rétention inférieure à 5,0 cm³/g environ et est capable de réguler le débit de liquide traversant.

2. Applicateur (30) suivant la revendication 1, caractérisé en outre en ce que ladite matière de dosage poreuse (6) est capable de limiter le débit de liquide traversant à une valeur comprise entre 0,25 et 10 ml/s environ.

3. Applicateur (30) suivant la revendication 2, caractérisé en outre en ce que la dimension moyenne de pore de ladite matière de dosage poreuse (6) est comprise entre 1 et 2000 µm environ et le volume de vide de ladite matière de dosage poreuse (6) est compris entre 10 et 70% environ.

4. Applicateur (30) suivant la revendication 2, caractérisé en outre en ce que ladite matière de dosage poreuse (6) est un polyéthylène haute densité ayant une dimension moyenne de pore comprise entre 60 et 100 µm environ et un volume de vide compris entre 40 et 60% environ.

5. Applicateur (30) suivant la revendication 1, caractérisé en outre en ce que ledit applicateur comprend en outre un réservoir (1) contenant la solution à appliquer par ledit applicateur, ce réservoir (1) étant contenu à l'intérieur dudit organe allongé creux (24) ou communiquant avec celui-ci.

6. Applicateur (30) suivant la revendication 5, dans lequel ledit organe allongé creux (24) comprend un deuxième orifice principal (11), à l'extrémité opposée audit premier orifice principal (20), et des moyens de fixation (4) formés sur l'intérieur dudit organe allongé creux (24) près dudit deuxième orifice principal (11), lesdits moyens de fixation (4) pouvant retenir ledit réservoir (11) ; et une collerette (12) radialement en saillie dans l'intérieur dudit organe allongé creux (24), ladite collerette (5) étant située près desdits moyens de fixation (4) et séparée dudit deuxième orifice principal (11) par lesdits moyens de fixation (4) ; et dans lequel ledit évent (9) comprend une rainure en forme de L dont une branche est creusée à travers lesdits moyens de fixation (4) et l'autre branche est creusée à travers une partie supérieure de ladite collerette (12).

7. Applicateur (30) suivant la revendication 1, dans lequel ladite matière spongieuse (8) a un rapport de fixation en compression de 1,5 à 10 environ, et une porosité comprise entre 10 et 100 pores environ par longueur de 2,54 cm.

FIG.1

FIG.2

FIG.3

FIG.4

FIG.5